(19) Europäisches Patentamt
European Patent Office
Office européen des brevets

(11) Veröffentlichungsnummer: **0 036 094**
**A1**

(12) **EUROPÄISCHE PATENTANMELDUNG**

(21) Anmeldenummer: **81101208.7**

(22) Anmeldetag: **20.02.81**

(51) Int. Cl.$^3$: **C 07 D 295/08**
A 61 K 31/40

(30) Priorität: **17.03.80 DE 3010152**

(43) Veröffentlichungstag der Anmeldung:
**23.09.81 Patentblatt 81/38**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28**
**D-8500 Nürnberg(DE)**

(72) Erfinder: **Liebenow, Walter, Dr. Dipl.-Chem.**
**Ganghoferstrasse 23**
**D-8500 Nürnberg 20(DE)**

(72) Erfinder: **Liedtke, Hans, Dr. Dipl.-Chem.**
**Chamer Strasse 37**
**D-8500 Nürnberg 30(DE)**

(74) Vertreter: **Kraus, Walter, Dr. et al,**
**Patentanwälte Dres. Kraus & Weisert Irmgardstrasse 15**
**D-8000 München 71(DE)**

(54) **Quaternäre 1,1-Diphenyl-4-pyrrolidinium-butin-(2)-salze, Verfahren zu ihrer Herstellung und diese Verbindungen enthaltendes Arzneimittel.**

(57) Es werden quaternäre 1,1-Diphenyl-4-pyrrolidinium-butin-(2)-salze der allgemeinen Formel

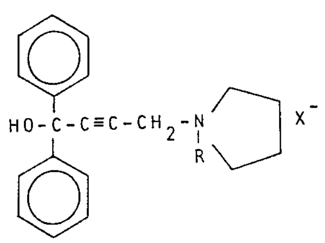

(1)

in der R für Methyl oder Äthyl steht und X ein Chlor- oder Bromatom bedeutet, beschrieben. Diese Verbindungen zeichnen sich durch eine verbesserte spasmolytische und broncholytische Aktivität aus.

EP 0 036 094 A1

Croydon Printing Company Ltd.

# Beschreibung

Die Erfindung betrifft quaternäre 1,1-Diphenyl-4-pyrrolidinium-butin-(2)-salze der allgemeinen Formel

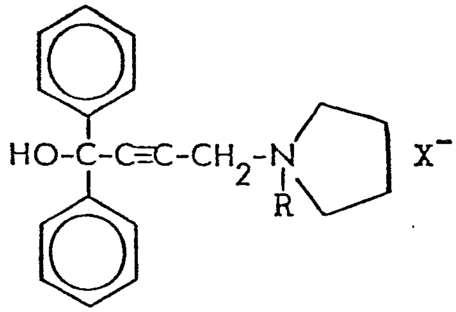

ein Verfahren zu ihrer Herstellung und diese Verbindungen enthaltende Arzneimittel.

Es ist bekannt, daß das Phosphat von 1,1-Diphenyl-4-pyrrolidino-2-butin-1-ol (Butinolin) mit der Strukturformel

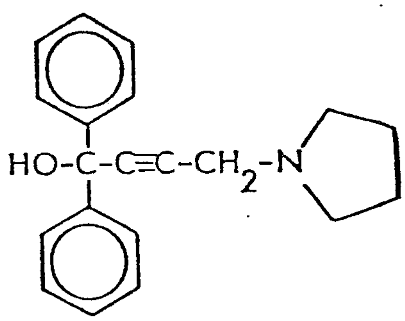

pharmakologisch aktiv ist und beispielsweise anticholinergische, spasmolytische und antiulcerogene Wirkungen hat. Diese Eigenschaften haben dazu geführt, daß bereits Arzneimittel auf dem Markt sind, welche Butinolinphosphat als Wirkstoff enthalten.

Es wurde nun überraschenderweise gefunden, daß quaternäre Butinolinsalze der oben angegebenen Formel eine bessere pharmakologische Aktivität besitzen als das bekannte Butinolinphosphat.

Gegenstand der Erfindung sind daher quaternäre 1,1-Diphenyl-4-pyrrolidinium-butin-(2)-salze der allgemeinen Formel

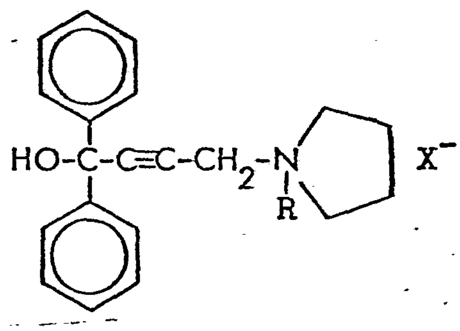

in der R für Methyl oder Äthyl steht und X ein Chlor- oder Bromatom bedeutet.

Diese Salze sind neue Verbindungen. Gegenüber dem bekannten Butinolinphosphat besitzen sie beispielsweise eine verbesserte spasmolytische und broncholytische Aktivität.

Aufgrund ihrer pharmakologischen Eigenschaften wird die Verbindung 1,1-Diphenyl-4-pyrrolidinyl-butin-(2)-ol-(1)-N-methyl-ammoniumbromid bevorzugt.

Ein weiterer Gegenstand der Erfindung ist ein Verfahren zur Herstellung dieser quaternären Salze, das dadurch gekennzeichnet ist, daß man in an sich bekannter Weise 1,1-Diphenyl-4-pyrrolidino-(2)-butin-1-ol der Formel

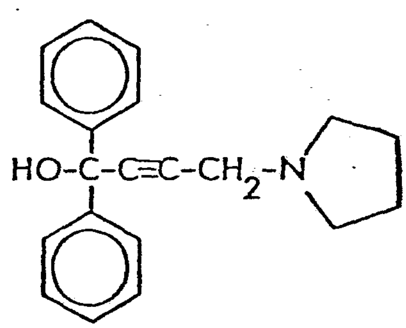

mit einem Alkylierungsmittel der Formel

R-X

in der R für Methyl oder Äthyl steht und X ein Chlor- oder Bromatom bedeutet, in einem aprotischen oder protischen Lösungsmittel zu dem entsprechenden Salz der Formel I umsetzt.

Bei der Umsetzung geht man von der bekannten Verbindung Butinolin aus, deren Herstellung von J.A. Gautier und C.C. Farnoux in Bull. Soc. Chim. France 1964, Seite 2147 beschrieben wird.

Die Quaternisierung der Butinolinbase erfolgt nach üblichen Methoden, wie sie zum Beispiel in "Organikum, Org.-chemisches Grundpraktikum", Autorenkollektiv, VEB Deutscher Verlag der Wissenschaften, Berlin 1977, Seite 260 beschrieben werden. Als Lösungsmittel für die Quaternisierung sind sowohl protische als auch aprotische Lösungsmittel geeignet. Beispiele hierfür sind Äthanol, Nitromethan, Acetonitril und Dimethylformamid. Zweckmäßig wird die Umsetzung mit einem Molverhältnis von Butinolinbase zu Alkylhalogenid von 1:1 bis 1:6 , vorzugsweise 1:2, durchgeführt. Vorzugsweise wird die Reaktion in einem Autoklaven unter einem Überdruck von 0,5 bis 5 bar, vorzugsweise 2 bar , durchgeführt. Die Reaktionstemperatur beträgt 65 bis 75 °C, vorzugsweise 70°C. Die Umsetzung ist gewöhnlich innerhalb eines Zeitraums von 5 bis 8 h , üblicherweise 7 h , beendigt. Die Aufarbeitung des Reaktionsgemisches erfolgt durch bekannte Methoden, beispielsweise durch Abdestillation des Lösungsmittels, gegebenenfalls im Vakuum, Zugabe eines organischen Lösungsmittels und Ausfällung mit einem hiermit mischbaren organischen Lösungsmittel. Die Reinigung erfolgt ebenfalls in bekannter Weise, beispielsweise durch Umkri-

0036094

stallisation aus einem organischen Lösungsmittel, wie Äthanol, bzw. durch Umfällung.

Wie oben bereits zum Ausdruck gebracht wurde, haben die erfindungsgemäßen Substanzen gegenüber dem bekannten Butinolinphosphat bessere pharmakologische Eigenschaften, insbesondere hinsichtlich der hiermit erzielbaren broncholytischen und antiulcerogenen Wirkung. Versuche bezüglich der Hemmung der durch Carbachol induzierten Speichelsekretion und der Mydriase haben ergeben, daß die erfindungsgemäßen Substanzen weniger Nebenwirkungen haben als das bekannte Butinolinphosphat.

In der nachstehenden Tabelle sind die Ergebnisse von pharmakologischen Untersuchungen zusammengestellt, die unter Verwendung der erfindungsgemäßen Substanz 1,1-Diphenyl-4-pyrrolidinyl-butin-(2)-ol-(1)-N-methyl-ammoniumbromid und von Butinolinphosphat als Vergleichssubstanz durchgeführt wurden.

Tabelle

| | 1,1-Diphenyl-4-pyrrolidinyl-butin-(2)-ol-(1)-N-methyl-ammoniumbromid | Butinolinphosphat | Test-Methode (Literatur-Ang.) |
|---|---|---|---|
| Broncholyse (Meerschweinchen) | $ED_{50}$ (µg/kg i.v.)<br>ACH ~ 30<br>Hi ~ 500 | $ED_{50}$ (µg/kg i.v.)<br>ACH ~ 60<br>Hi ~ 1000 | Konzett, H., Rössler, R., Arch.exp.Pharmacol.Path., <u>195</u>, 71 (1940) |
| Antiulcerogene Wirkung Streßulcus | $ED_{50}$ (mg/kg)<br>i.g.<br>25 | $ED_{50}$ (mg/kg)<br>i.g.<br>150 | Takagi, K., Okabe, S., Jap. J.Pharmac. <u>18</u>, 9 (1968) |
| Hemmung der durch Carbachol induzierten Speichelsekretion (Ratte) cholinolytische Nebenwirkungen | $ED_{50}$ (mg/kg)<br>i.g.<br>24 | $ED_{50}$ (mg/kg)<br>i.g.<br>9,8 | mod. nach Brown, D.M., Quinton, R.M., Brit.J.Pharmacol. <u>12</u>, 53 (1957) |

Tabelle (Forts.)

| | 1,1-Diphenyl-4-pyrrolidinyl-butin-(2)-ol-(1)-N-methyl-ammonium bromid | Butinolin-phosphat | Test-Methode (Literatur-Ang.) |
|---|---|---|---|
| Mydriase cholinolytische Nebenwirkungen | $ED_{50}$ (mg/kg) i.g. 128 | $ED_{50}$ (mg/kg) i.g. 16 | Pulewka, P.Arch. exp. Pharmacol. Path. 168, 307 (1932) |
| Spasmolytische Wirkung in vitro (Mee-Ileum) | $pA_2$ (ACH) 8,0 | $pA_2$ (ACH) 8,2 | Van Rossum, J.M., Arch.Int.Pharmacodyn.Ther. 143, 299 (1963) |
| Hemmung der durch Carbachol induzierten Magensekretion (Heidenhain-Katze) | $ED_{50}$ (mg/kg) i.g. 0,3 | $ED_{50}$ (ACH) i.g. 0,3 | Engler, H., Fritschi, E., Naunyn Schmiedeberg's Arch.Pharmacol. 297, R 54 (1977) |

Ein weiterer Gegenstand der Erfindung ist daher ein Arzneimittel, insbesondere Spasmolytikum und Antiulcusmittel, das dadurch gekennzeichnet ist, daß es neben üblichen Hilfs- und Trägerstoffen ein quaternäres Salz der oben angegebenen Art enthält.

Die erfindungsgemäßen Arzneimittel können als pharmazeutische Präparate mit direkter oder verzögerter Freigabe des Wirkstoffs in Mischung mit einem für die orale oder parenterale Applikation geeigneten organischen Trägermaterial oder anorganischen inerten Trägermaterial, zum Beispiel Calciumhydrogenphosphat, Cellulose, Dextrose, Maisstärke, Saccharose, Magnesiumstearat, Lactose, Gelatine, Polyvinylpyrrolidin, pflanzlichen Ölen, Polyäthylenglykol, Vaseline usw., formuliert werden. Das erfindungsgemäße Arzneimittel kann in fester Form, beispielsweise als Tablette oder Kapsel, als Lösung, Suspension oder Emulsion oder in Form von Suppositorien vorliegen. Weiterhin kann das erfindungsgemäße Arzneimittel den Wirkstoff einer an sich bekannten Teezubereitung bilden. Die Einzeldosis, bezogen auf den Wirkstoff, beträgt bei peroraler Applikation 5 bis 50 mg, vorzugsweise 10 bis 30 mg. Die Tagesdosis kann in einer oder mehreren Einzeldosen verabreicht werden.

Die Erfindung wird in dem folgenden Beispiel erläutert.

Beispiel

Herstellung von 1,1-Diphenyl-4-pyrrolidinyl-butin-(2)-ol-(1)-N-methyl-ammoniumbromid

Man legt 29,1 g (0,1 Mol) 1,1-Diphenyl-4-pyrrolidino-(2)-butin-1-ol in einem 0,5-l-Labor-Autoklaven vor, suspendiert unter Rühren in 140 ml Dimethylformamid und kühlt mit Aceton-Trockeneis auf ca. -40°C ab. Danach werden 19,0 g (0,2 Mol) Methylbromid zugegeben, und der Autoklav wird geschlossen.

Nach 7 Stunden Erhitzen auf 70°C unter Rühren läßt man erkalten und dampft das Dimethylformamid im Wasserstrahlpumpenvakuum am Rotationsverdampfer ab. Darauf werden 30 ml 99,7%-iger Äthanol hinzugegeben und auf dem 60°C heißen Wasserbad gelöst. Man fügt 50 ml Essigester langsam unter Rühren hinzu und läßt zur Kristallisation stehen.

Nach dem Absaugen wird das Produkt durch Auflösen in 50 ml 99,7%igem Äthanol unter Erwärmen, Abkühlen auf +10°C und Zugabe von 60 ml Essigester und 30 ml Diäthyläther umkristallisiert. Man saugt ab und trocknet an der Luft.

Dünnschichtchromatographie (100 % $CH_3OH$) 1 Fleck
Schmelzpunkt: 147,8 - 148,7°C
Ausbeute: 30,6 g ≙ 79,2 % d.Th.

KRAUS & WEISERT
PATENTANWÄLTE
DR. WALTER KRAUS DIPLOMCHEMIKER · DR.-ING. ANNEKÄTE WEISERT DIPL.-ING. FACHRICHTUNG CHEMIE
IRMGARDSTRASSE 15 · D-8000 MÜNCHEN 71 · TELEFON 089/797077-797078 · TELEX 05-212156 kpat d
TELEGRAMM KRAUSPATENT

LUDWIG HEUMANN & CO GMBH

Nürnberg

---

Quaternäre 1,1-Diphenyl-4-pyrrolidinium-butin-(2)-salze, Verfahren zu ihrer Herstellung und diese Verbindungen enthaltendes Arzneimittel

---

## Patentansprüche

1. Quaternäre 1,1-Diphenyl-4-pyrrolidinium-butin-(2)-salze der allgemeinen Formel

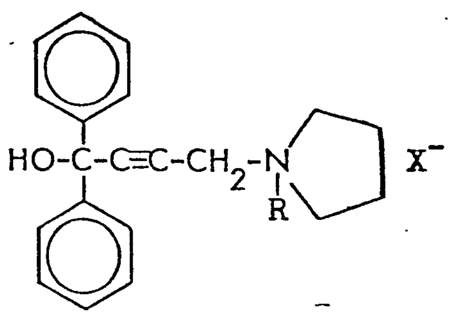

(I)

in der R für Methyl oder Äthyl steht und X ein Chlor- oder Bromatom bedeutet.

2. 1,1-Diphenyl-4-pyrrolidinyl-butin-(2)-ol-(1)-N-methyl-ammoniumbromid.

3. Verfahren zur Herstellung von quaternären 1,1-Diphenyl-4-pyrrolidinium-butin-(2)-salzen nach Anspruch 1 oder 2, dadurch gekennzeichnet, daß man in an sich bekannter Weise 1,1-Diphenyl-4-pyrrolidino-(2)-butin-1-ol der Formel II

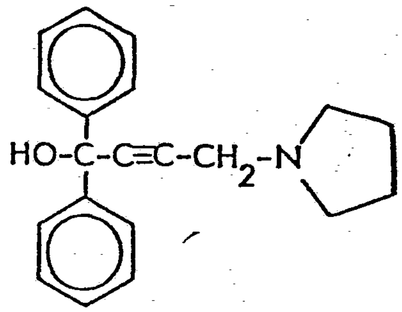

(II)

mit einem Alkylierungsmittel der Formel III

R-X (III)

in der R für Methyl oder Äthyl steht und X ein Chlor- oder Bromatom bedeutet, in einem aprotischen oder protischen Lösungsmittel zu dem entsprechenden Salz der Formel I umsetzt.

4. Arzneimittel, insbesondere Spasmolytikum und Antiulcusmittel, dadurch gekennzeichnet, daß es neben üblichen Hilfs- und Trägerstoffen eine Verbindung nach Anspruch 1 enthält.

Patentanspruch für Österreich

P a t e n t a n s p r u c h

Verfahren zur Herstellung von quaternären 1,1-Diphenyl-4-pyrrolidinium-butin-(2)-salzen der allgemeinen Formel I

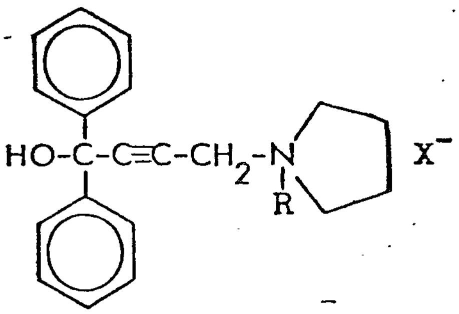

(I)

in der R für Methyl oder Äthyl steht und X ein Chlor- oder Bromatom bedeutet,

dadurch g e k e n n z e i c h n e t, daß man in an sich bekannter Weise 1,1-Diphenyl-4-pyrrolidino-(2)-butin-1-ol der Formel II

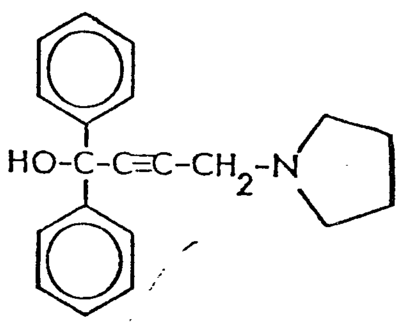

(II)

mit einem Alkylierungsmittel der Formel III

R-X (III)

in der R für Methyl oder Äthyl steht und X ein Chlor- oder Bromatom bedeutet, in einem aprotischen oder protischen Lösungsmittel zu dem entsprechenden Salz der Formel I umsetzt.

Europäisches Patentamt

# EUROPÄISCHER RECHERCHENBERICHT

0036094

Nummer der Anmeldung

EP 81101208.7

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | M. NEGWER "Organisch-chemische Arzneimittel und ihre Synonyma" 5. Auflage, Band II, 1978, AKADEMIE-VERLAG Berlin, Seite 771, Spalte 1, laufende Nr. 4217 | 1-4 | C 07 D 295/08<br>A 61 K 31/40 |
| | -- | | |
| | FR - M - 2 142 (LAKESIDE LABORATORIES)<br>+ Patentanspruch 1 +<br>---- | 1,4 | |

RECHERCHIERTE SACHGEBIETE (Int. Cl.³)

C 07 D 295/00
C 07 D 207/00
A 61 K 31/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 16-06-1981 | ONDER |